# EUROPEAN PATENT APPLICATION

(11) **EP 4 166 223 A1**
(43) Date of publication of application: **19.04.2023**
(21) Application number: 21202682.7
(22) Date of filing: 14.10.2021
(51) Int. Cl.: B01D 61/16, B01D 61/20, C07K 1/14, C07K 1/34, C07K 1/36, B01D 17/02, B01D 11/04, C07K 16/00

(54) **SEPARATION SYSTEM AND METHOD FOR SEPARATING AND PURIFYING A TARGET COMPONENT**

(71) Applicant: Sartorius Stedim Biotech GmbH, 37079 Göttingen (DE)
(72) Inventor: Kruse, Thomas, Göttingen (DE); Kampmann, Markus, Dortmund (DE); Helling, Alexander, Ebergötzen (DE); Leuthold, Martin, Göttingen (DE)
(74) Representative: Müller-Boré & Partner Patentanwälte PartG mbB

(57) **Abstract**

The present invention relates to a separation system for separating and purifying a target component, a method for separating and purifying a target component, and the use of a crossflow diafiltration unit for processing a target phase, which is obtained after separation of an aqueous two-phase system and contains a target component.

## Description

The present invention relates to a separation system for separating and purifying a target component, a method for separating and purifying a target component, and the use of a crossflow diafiltration unit for processing a target phase, which is obtained after separation of an aqueous two-phase system and contains a target component.

In recent years, there have been achieved large improvements in the upstream process of target component producing cell lines. This led to the situation that the bottleneck in production was shifted to the subsequent clarification, capture and purification unit operations in the downstream process (DSP).

One example are monoclonal antibodies (mAb). Due to their importance for the treatment of several severe diseases like cancer, autoimmune disorders and inflammatory diseases, the market for monoclonal antibodies rises. Ongoing improvements in the upstream process of mAb producing cell lines, like Chinese hamster ovary (CHO) cells, have resulted in increased cell densities and high product titers up to 25 g/l. However, these achievements shifted the bottleneck in mAb production to the DSP.

As mAb are secreted into the medium, cells must be removed first. Most commonly continuous disk-stack centrifuges are used as a first clarification step at commercial scale due to their economic benefits and scalability. Alternative clarification methods are depth filtration, flocculation, dynamic body feed filtration or the usage of an acoustic cell retention device. As subsequent mAb capture step, protein A affinity chromatography is used in most platform processes, offering the advantages of high yield and purity as well as a volume reduction. However, challenges in DSP are mainly caused by the limited capacity of chromatographic methods and, especially for protein A chromatography, its price.

A promising approach to overcome this bottleneck is the application of aqueous two-phase extraction (ATPE; Figure 1) as described in WO 2014/135420 A1 and US 2011/0257378 A1. Formation of aqueous two-phase systems (ATPS) takes place by mixing a variety of phase forming components in water. Above a certain concentration of the phase forming components, two immiscible phases are formed. As phase forming components, polymer/polymer (usually polyethylene glycol (PEG) and dextran) or polymer/salt (e.g., phosphate, citrate or sulfate) are most commonly used. In the latter case, for example, a light, polymer rich (LP) and a heavy salt rich phase (HP) are formed. Based on the composition of the ATPS, a selective extraction of the target molecule (e.g. mAb) can be achieved, while impurities like deoxyribonucleic acid (DNA) and host cell proteins (HCP) are enriched in the other phase. Particles like cells, cell debris and other bioparticles accumulate at the ATPS interphase enabling an integration of clarification and first target component purification.

It has been demonstrated that ATPE is suitable as a first purification step in the downstream process of mAb, has economic benefits and is environmentally sustainable compared to current established platform processes (cf. Kruse et al. Antibodies 2019, 8, 40; and Kruse et al. Biochemical Engineering Journal, Volume 161, 107703). For further purification, a separation of the mAb containing target phase must be ensured. Established methods make use of the different densities of both phases from an ATPS.

Phase separation by gravity is often conducted by mixer-settler devices. However, due to the minor density difference between both phases this method is often time consuming and thus expensive. Alternatives heretofore are column or centrifugal extractors where the separation is also realized by means of density differences between both phases. However, these methods require an additional sterile filtration step if used as clarification unit operation to ensure complete cell removal. Membrane technology like crossflow diafiltration has also been applied for phase separation (cf. Kruse et al. Antibodies 2019, 8, 40; and Kruse et al. Biochemical Engineering Journal, Volume 161, 107703).

For subsequent purification steps, it is commonly necessary to conduct a buffer exchange. For this purpose, the separated, target containing phase can be further processed by diafiltration. However, such membrane technology suffers from precipitation of the target components and, thus, high loss of product or the need to apply a dilution step before filtration and, thus, generates more waste, expenditure of time and expenses.

Thus, the technical problem underlying the present invention is to provide means for downstream processing of target components, whereby said means should be rather inexpensive and should still provide high purities and yields when compared to conventional means for DSP. In particular, when applying ATPE as a purification step, it is aimed at providing means for efficient buffer exchange for subsequent purification steps.

The solution to the above technical problem is achieved by the embodiments characterized in the claims.

In particular, the present invention relates to a separation system configured to process a fluid containing a plurality of components, wherein at least one component of the plurality of components of the fluid is a target component and wherein the separation system comprises:
an aqueous-two phase extraction unit for aqueous-two phase extraction of the fluid,
a separation unit for separating a target phase obtained after aqueous-two phase extraction, wherein the target phase contains the target component, and
a crossflow diafiltration unit for continuous diafiltration of the separated target phase for obtaining a retentate and a permeate, wherein the crossflow diafiltration unit at least comprises a diafiltration channel, a first filter material, a retentate channel, a second filter material and a permeate collection channel, arranged in such a way that the first filter material delimits the diafiltration channel and the retentate channel from one another, and the second filter material delimits the retentate channel and the permeate collection channel from one another,
wherein the diafiltration channel is connected in a fluid conducting manner to at least one inlet for the diafiltration medium; the retentate channel is connected in a fluid conducting manner to at least one inlet for the target phase and to at least one outlet for the retentate;
and the permeate collection channel is connected in a fluid conducting manner to at least one outlet for the permeate.

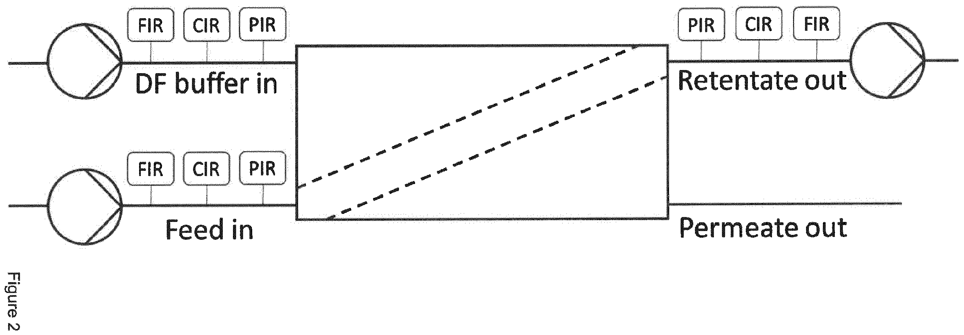

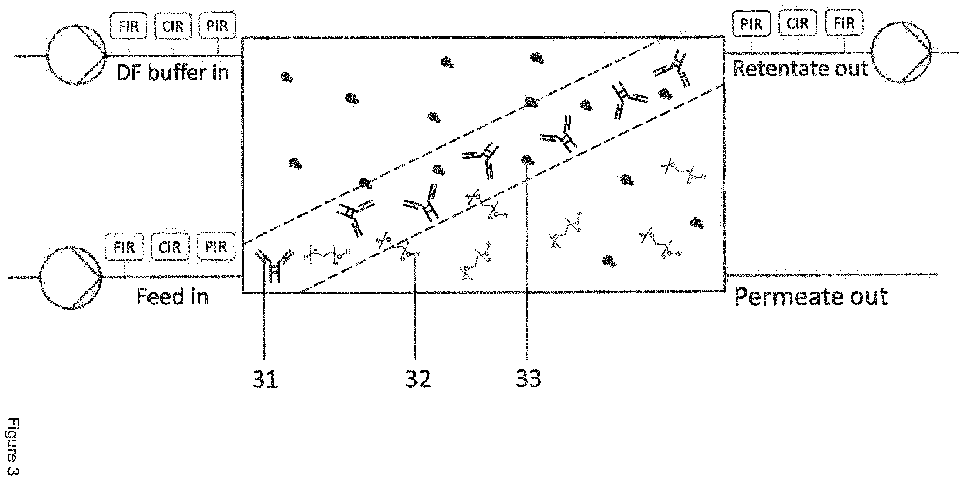

With the separation system of the present invention, it is preferably advantageously possible to directly apply the target phase obtained after separation from an aqueous two-phase extraction to crossflow diafiltration without having to previously dilute the target phase. Thereby, the necessary amounts of the liquid can significantly be reduced. Moreover, precipitation of target component during diafiltration can preferably advantageously be avoided by applying the specific crossflow diafiltration unit (cf. Figures 2 and 3), which uses the target phase as feed fluid. Furthermore, preferably high yields of the target component in high concentration can advantageously be achieved after diafiltration and buffer exchange can be carried out very quickly.

The target component is not specifically limited. For example, the target component may be any biomolecule of interest, such as proteins, mAbs, hormones, vaccines, nucleic acids, and exosomes, and viruses, and virus-like particles. In a preferred embodiment, the target component is an antibody, more preferably a monoclonal antibody, or fragments or derivatives thereof. Examples of monoclonal antibodies are adalimumab, cetuximab, rituximab, infliximab, omalizumab, and denosumab. The target component can be obtained, for example, from cell cultures, such as "Chinese Hamster Ovary Cells" (CHO cells) or other mammalian cell lines. The fluid as starting material for ATPE is not specifically limited as long as it contains the target component. Suitable fluids are cell broth and cell broth supernatant obtained by filtration or centrifugation methods or further purified material.

The aqueous-two phase extraction unit used in the present invention is not specifically limited. For example, any vessel, container, etc., which is able to host an aqueous two-phase system can be applied.

The term "two-phase system" is not subject to any particular limitations as long as the composition is capable of forming at least two phases. Preferably, the composition forms two phases. Commonly, the upper phase thereby has a lower density than the lower phase(s).

Any ATPS can be used. Moreover, any phase of the ATPS can be subjected to diafiltration. Examples of ATPS are (1) polymer-polymer systems (e.g. PEG/dextran); (2) polymer-salt systems (e.g. PEG/phosphate or citrate or sulfate salts); (3) alcohol-salt systems (e.g. propanol or ethanol/salt); (4) micellar ATPS (based on surfactants); and (5) ATPS based on ionic liquids.

The target component can be in any phase of the ATPS. Preferably, the target component is mainly in one of the phases of the ATPS. Preferably, the desired substance is mainly in the upper phase.

The composition forms a two-phase system in which the target component is preferentially located in one of the phases. The proportion of the target component, based on the total amount/total mass of the target component in the composition, in the enriched phase is not subject to any particular limitations. The proportion of the target component, such as the antibody, in the enriched phase is, for example, at least 70% by weight, at least 80% by weight, at least 90% by weight, at least 95% by weight, at least 97% by weight, at least 98% by weight, or at least 99% by weight, based on the total mass of the target component in the ATPS.

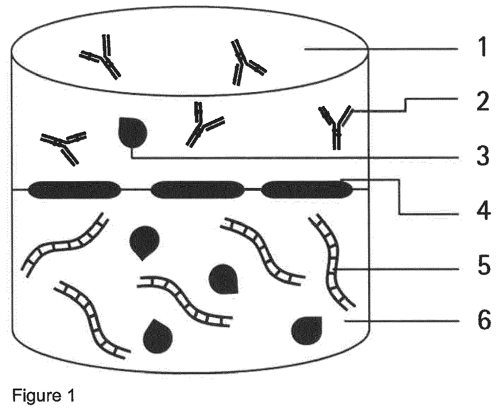

Due to different biophysical properties of the components subjected to ATPE, they distribute differently between the two phases (Figure 1). This distribution coefficient can be influenced by the composition of the aqueous two-phase system. Further parameters for influencing that distribution coefficient are for example temperature, pH, and properties of the target components. For each process/product, the composition of the aqueous two-phase system can be redetermined for optimum purity and yield.

The optimization of the ATPS composition with a high yield and purity of the target molecule has already been described in many studies using a statistical design of experiments (DoE) approach (Gronemeyer, P. et al. Biochem. Eng. J. 2016, 113, 158-166; Glyk, A. et al. Chemom. Intell. Lab. Syst. 2015, 149, 12-21; Rosa, P.A.J. et al. J. Chromatogr. A 2007, 1141, 50-60; Kruse et al. Antibodies 2019, 8, 40; and Kruse et al. Biochemical Engineering Journal, Volume 161, 107703). For a given target component and accompanying impurities, there can be created a model which can then be used to derive an optimum system composition with the greatest possible yield of the target component and at the same time the greatest reduction in process-related impurities.

For example, there can be applied a composition for an aqueous two-phase system comprising, based on the total amount of the composition, from 4.0 to 25.0 wt.% of polyethylene glycol having an average molecular weight of from 300 to 20000; from 5.0 to 40 wt.% first salt selected from a phosphate salt, a citrate salt and a sulfate salt, or polymer, in particular dextran; 0 to 10.0 wt.% second salt, in particular a chloride salt or diammonium sulfate (NH₄)₂SO₄; 10.0 to 50.0 wt.% fluid containing the target component, such as cell culture broth or a filtrate thereof; and the balance being water. Appropriate other systems are known to a person skilled in the art. The proportion of the individual components is selected in such a way that an ATPS forms. The average molecular weight and the content of polyethylene glycol are appropriately chosen with respect to each other. For example, when applying polyethylene glycol with an average molecular weight at the upper end, the content of the same may be chosen at the lower end and *vice versa.*

The indicated amounts of the different components refer to the total amount/total mass of the composition. In addition to polyethylene glycol, first salt or polymer, second salt and the fluid (e.g. cell culture broth or a filtrate thereof), other components may be included, as known in the prior art. In this case, the residual amount remaining after addition of the other components except water corresponds to the amount of water contained in the composition, wherein water contained in the fluid (e.g. cell culture broth or filtrate) is included in the amount of fluid, and thus not in the water residual. The components may be used in the form of pure substances or in solution to form the composition. The indicated proportions by weight of these components in the composition correspond to the proportions by weight of the corresponding pure substances. The proportions of any solvents are thus added to the optional further components or, in the case of aqueous solutions, to the water. For example, a composition with 1 kg total mass, prepared by using, among other things, 600 g of a 40% (w/w) aqueous phosphate salt solution and 90 g of a 50% (w/w) aqueous PEG1450 solution, has 24 wt.% (240 g) phosphate salt and 4.5 wt.% (45 g) PEG1450.

The term "polyethylene glycol" is not specifically limited. A single polyethylene glycol or a mixture of several polyethylene glycols with different molecular weights may be used.

The composition may comprise polyethylene glycol having an average molecular weight of 300 to 20000. Preferably, the polyethylene glycol has an average molecular weight of 350 to 10000, more preferably 350 to 4000, more preferably 400 to 2000. Corresponding polyethylene glycols are commercially available, for example.

The pH of the composition for ATPE is not subject to any particular limitations and may be, for example, from 4.0 to 10.0, preferably from 5.0 to 9.0, more preferably from 6.0 to 8.0. At pH values higher than 8.0, deamination may occur in the amino acids of, for example, an antibody.

The duration for ATPE is not particularly limited. Preferably, ATPE is carried out until phase equilibrium is reached.

The separation unit used in the present invention is not specifically limited. Thus, any separation unit known in the art for separating phases obtained after aqueous-two phase extraction can be applied. Suitable separation units include for example mixer-settler devices, centrifuges and column or centrifugal extractors as well as membrane-based phase separation methods (cf. Kruse et al. Antibodies 2019, 8, 40). Preferably, the separation unit is a centrifuge. By means of the separation unit, it is possible to separate the phases obtained after ATPE from each other. The target phase containing (the majority of) the target component can then be transferred to the diafiltration unit.

The crossflow diafiltration unit at least comprises a diafiltration channel, a (preferably flat) first filter material, a retentate channel, a (preferably flat) second filter material and a permeate collection channel, arranged in such a way that the first filter material delimits the diafiltration channel and the retentate channel from one another, and the second filter material delimits the retentate channel and the permeate collection channel from one another,
wherein the diafiltration channel is connected in a fluid conducting (communicating) manner to at least one inlet for the diafiltration medium; the retentate channel is connected in a fluid conducting manner to at least one inlet for the target phase and to at least one outlet for the retentate; and the permeate collection channel is connected in a fluid conducting manner to at least one outlet for the permeate. An example of a respective crossflow diafiltration unit is for example schematically shown in Figures 2 and 3. Suitable crossflow diafiltration units are e.g. described in DE 10 2016 004 115 A1 (crossflow diafiltration unit for continuous diafiltration).

The shape (spatial form) of the first and second filter materials is not subject to any particular restriction. Preferably, the first filter material and the second filter material are flat filter materials. The term "flat" indicates that the respective filter material lies substantially in a single plane. Preferably all filter materials lie more or less in planes that are substantially parallel to each other.

In addition, the crossflow diafiltration unit can be a wound module. For this purpose, an initially flat or planar arrangement of first and second filter material is wound around a core, so that first and second filter material (as well as diafiltration, retentate and permeate channels) each have a spiral cross-section.

In addition, the crossflow diafiltration unit may be a hollow fiber module. In this case, the first and second filter materials have a hollow fiber shape (cylinders without circular disk-shaped end sections). The inner diameters of the first and second hollow fiber filter materials are different to allow the first and second filter materials to be inserted into the second and first filter materials, respectively, to form the diafiltration, retentate, and permeate channels.

The first filter material has preferably a molecular weight cut-off (MWCO) in the range of 1 kDa to 1,500 kDa. The second filter material has preferably a molecular weight cutoff in the range of 1 kDa to 1,500 kDa. The determination of the molecular weight cut-off can be carried out in accordance with the US standard ASTM E1343-90 ("Standard test method for molecular weight cutoff evaluation of flat sheet ultrafiltration membranes"). The MWCO of the membranes is preferably selected such that the target component is retained in the retentate channel, while the smaller phase forming components of the ATPS permeate through the membrane and are withdrawn in the permeate outlet.

The first filter material has preferably a pore size of 0.001 to 50 µm, preferably 0.01 to 0.5 µm. The second filter material has preferably a pore size of 0.001 to 50 µm, preferably 0.01 to 0.5 µm.

For pore sizes of at least 0.1 µm, i.e. for microfiltration membranes with an average pore size of 0.1 to 10 µm, capillary flux porometry is used to determine the pore size. This is a gas/liquid porosimetry in which the differential gas pressures and flow rates through a membrane sample are measured first in the wet and then in the dry state. Prior to measurement, the membrane sample is brought into contact with a wetting liquid in such a way that all pores are filled with this liquid. After the pores have been filled and the sample has been introduced, the measuring cell must be closed and the measurement started. After starting the measurement, the gas pressure is automatically and gradually increased and the pore diameters corresponding to the applied pressure are emptied by the gas pressure. This is continued until the relevant pore range has been covered, i.e. until even the smallest pores present in the measuring range have been freed of liquid. The pressure is then reduced again and the measurement is repeated automatically on the now dry sample. The pore size distribution is calculated from the difference between the two pressure-flow rate curves using the Young-Laplace equation (see also A. Shrestha, "Characterization of porous membranes via porometry", 2012, Mechanical Engineering Graduate Theses & Dissertations, Paper 38, University of Colorado at Boulder).

For the determination of pore sizes larger than 10 µm and up to 1 mm, the image analysis based method described in Journal of Membrane Science 372 (2011), pages 66 to 74, can be used.

For pore sizes less than 0.1 µm, the liquid-liquid displacement method, which has similarities to capillary flow porometry, is used. However, instead of the gas flow rates, the flow rates of the displacing liquid are measured as a function of the differential pressure increase (see also R. Dávila, "Characterization of ultra and nanofiltration commercial filters by liquid-liquid displacement porosimetry", 2013).

According to a preferred embodiment, the first filter material is a first filtration membrane or the second filter material is a second filtration membrane. It is particularly preferred that the first filter material is a first filtration membrane and that the second filter material is a second filtration membrane.

In particular, a porous membrane in the ultrafiltration and microfiltration range is suitable as the first filter material and as the second filter material.

In a preferred embodiment, the first filter material and the second filter material are identical.

The ultrafiltration membranes are characterized by pore sizes of less than 0.01 µm or by molecular weight cut-offs that are approximately in the molecular weight range of 1 to 1,500 kDa, while the microfiltration membranes have pore sizes in the range of 0.01 to 50 µm, preferably 0.01 to 0.5 µm, or molecular weight cut-offs of 30 to 1,500 kDa. The filtration membranes may consist, for example, of polyvinylidene fluoride, cellulose and derivatives thereof, polyethersulfone or polysulfone, with crosslinked cellulose hydrate being particularly preferred.

The inlet for the feed fluid (here: target phase) is mounted preferably in a first edge region of the crossflow diafiltration unit; and the outlet for the retentate is mounted in a second edge region of the crossflow diafiltration unit that faces the first edge region. Owing to this arrangement it is possible to define a substantially uniform direction of flow of the retentate from the inlet for the feed fluid, as the starting point, to the outlet of the retentate, as an end point. As a result, the direction of flow of the retentate runs substantially parallel to the flow path along the (flat) filter material, i.e., in essence without deflections, so that a stable and reliable flow of the retentate can be ensured by the crossflow diafiltration unit. In addition, due to the substantially linear flow path without deflections, loops or the like, it is possible to minimize the pressure drop in the filtration unit as well as undesired effects of non-linear flows on the target substances, contained in the feed fluid. For the above reasons it is also preferred that the inlet for the diafiltration medium be mounted in the first edge region of the crossflow diafiltration unit. However, it is also possible to mount the inlet for the diafiltration medium in the second edge region or in the third and/or fourth edge region.

According to a preferred embodiment, the outlet for the permeate is mounted in the second edge region of the crossflow diafiltration unit. It is particularly preferred that in each case at least one outlet for the permeate be mounted in both the first and the second edge region of the crossflow diafiltration unit. In another embodiment of the invention the outlets for the permeate are mounted, as an alternative or in addition, in the third and/or fourth edge region of the crossflow diafiltration unit. The third edge region is located on the left side of the flow direction in a plan view of the crossflow diafiltration unit from the side of the diafiltration channel. Correspondingly the fourth edge region is located on the right side and, thus, lies opposite the third edge region. The above arrangement of the outlet(s) makes it possible to achieve not only a particularly high permeate performance, but also design advantages.

The first edge region comprises preferably the outer third of the length of the filtration unit opposite to the direction of flow. Correspondingly the second edge region comprises the outer third of the length of the filtration unit along the direction of flow. The same applies to the third and fourth edge regions. It is advantageous to make the first to fourth edge regions as small as possible. Therefore, it is particularly preferred that the edge regions comprise the respective outer 20%, even more preferably the respective outer 10%, and most preferably the respective outer 3%.

In principle, there is no particular constraint on the mounting of the inlets and outlets. For example, the inlets and outlets may be mounted in such a way that the feed fluid already enters the retentate channel in the direction of flow and leaves it in the direction of flow. Correspondingly, the outlet for the permeate can be mounted in such a way that the permeate leaves the permeate collection channel in the direction of flow; and/or the inlet for the diafiltration medium can be mounted in such a way that it enters the diafiltration channel in the direction of flow. Preferably, however, the inlets and outlets are mounted in such a way that the diafiltration medium enters the diafiltration channel perpendicular to the direction of flow; and then the feed fluid enters the retentate channel perpendicular to the direction of flow and leaves it, as a retentate, perpendicular to the direction of flow.

Such a mounting of the inlets and outlets facilitates the arrangement of a plurality of the inventive filtration units to form a filter cassette. A respective arrangement is e.g. schematically shown in Figs. 2 and 3. As shown in Fig. 3, the ATPS target phase containing the target component as well as the phase forming components is supplied via the feed inlet. Due to the selected MWCO of the membrane, the target component is retained in the retentate channel, while the smaller phase forming components permeate through the membrane and are withdrawn in the permeate outlet. Simultaneously, the diafiltration buffer is actively supplied by an additional channel, permeates through the membrane and washes out the phase forming components from the retentate to the permeate (buffer exchange).

Preferably the crossflow diafiltration unit comprises a plurality of inlets for the feed fluid (here target phase), a plurality of outlets for the retentate, and a plurality of outlets for the permeate.

In continuous diafiltration both the feed fluid (here: target phase) and the diafiltration medium are added continuously so that the process does not have to be interrupted. As a result, the crossflow diafiltration unit makes it possible to run the process in an efficient and economical manner.

The diafiltration medium is not particularly limited. In principle, any fluid is suitable, with water and aqueous salt solutions being preferred. For example, an aqueous buffer solution can be used as the diafiltration medium. Preferably, the diafiltration medium is selected from the group consisting of KPi buffer, sodium phosphate buffer, sodium acetate buffer, PBS, glycine, citrate buffer, Tris buffer, BIS-Tris buffer, HEPES buffer, and water. The concentration of the buffer in the aqueous solution is not particularly limited and may for example be from 1.0 mM to 5.0 M, preferably from 5.0 mM to 1.0 M, more preferably from 10 mM to 200 mM, most preferably from 25 to 100 mM.

The pH of the diafiltration medium is not particularly limited. For example, pH of the diafiltration medium may be from 2.0 to 12.0, preferably from 3.0 to 10.0, most preferably, from 5.0 to 9.0. When using a diafiltration at low pH, preferably from 2.5 to 4.5, most preferably, from 3.0 to 4.0, it is preferably advantageously possible to achieve virus inactivation by acidification during diafiltration.

In a preferred embodiment, the crossflow diafiltration unit is configured such that a volume flow rate of the supplied diafiltration medium is 0.5 to 20 times a volume flow rate of the supplied target phase (i.e. the supplied feed fluid). The volume flow rate of the supplied diafiltration medium is preferably 1.0 to 15, more preferably 2.0 to 10, more preferably 4.0 to 9.0, most preferably 5.0 to 7.0 times the volume flow rate of the supplied target phase.

The volume flow rate of the supplied diafiltration medium can be from 1.0 mL/min to 2.0 L/min, preferably from 2.0 mL/min to 1.0 L/min, more preferably from 4.0 mL/min to 500 mL/min, most preferably from 10 mL/min to 175 mL/min. The volume flow rate of the supplied target phase can be from 0.5 mL/min to 100 mL/min, preferably from 1.0 mL/min to 50 mL/min, more preferably from 2.0 mL/min to 25 mL/min. The volume flow rate of the retentate at the outlet can be from 0.5 mL/min to 100 mL/min, preferably from 1.0 mL/min to 50 mL/min, more preferably from 2.0 mL/min to 25 mL/min.

In a preferred embodiment, the diafiltration medium is supplied at a pressure of 0.1 to 4 bar. More preferably, the diafiltration medium is supplied at a pressure that is greater than the retentate outlet pressure.

Preferably, diafiltration is carried out continuously, i.e., under constant/continuous addition of the diafiltration medium and the feed fluid, so that a particularly efficient and economical filtration method can be provided.

Preferably, the separated target phase is directly applied to the crossflow diafiltration unit. Accordingly, the separated target phase is preferably applied without any dilution to the crossflow diafiltration unit. If necessary, the separated target phase may be subjected to filtration (e.g., with a 0.2 µm sterile filter) prior to diafiltration. Potential washing steps may be carried out after said filtration to such an extent that no significant dilution occurs (e.g., a dilution with less than 0.50 volume equivalent, more preferably less than 0.1 volume equivalent, more preferably less than 0.01 volume equivalent).

The concentration of the target component in the separated target phase is not particularly limited. For example, the concentration of the target component in the separated target phase may be from 0.01 g/L to 500 g/L, preferably from 0.1 g/L to 50 g/L, most preferably, from 0.5 g/L to 10 g/L.

The concentration of the target component in the retentate after diafiltration is not particularly limited. For example, the concentration of the target component in the retentate after diafiltration may be from 0.01 g/L to 500 g/L, preferably from 0.1 g/L to 50 g/L, most preferably, from 0.5 g/L to 10 g/L.

After crossflow diafiltration, the (majority of the) target component is preferably contained in the retentate. Thus, the mass ratio of the mass of the target component included in the retentate to the total mass of the target component subjected to diafiltration is preferably more than 50 mass%, more preferably at least 80 mass%, more preferably at least 90 mass%, most preferably at least 95 mass%.

After diafiltration, the retentate or permeate, preferably retentate, can be subjected to further purification steps, such as affinity and/or hydrophobic interaction and/or cation and/or anion exchange chromatography and/or sterile filtration and/or virus filtration and/or virus inactivation in any suitable order. Accordingly, the separation system of the present invention can further comprise respective further purification units, such as affinity and/or hydrophobic interaction and/or cation and/or anion exchange chromatography units and/or sterile filtration and/or virus filtration units and/or virus inactivation units in any suitable order. Preferably, the diafiltration medium is chosen such that the resulting retentate or permeate, preferably retentate, has the appropriate buffer for the subsequent purification step(s).

In a further aspect, the present invention relates to a method for purifying a target component included in a fluid, wherein the method comprises the steps of
(a) subjecting the fluid to aqueous two-phase extraction,
(b) separating a target phase containing the target component from the other phase obtained after aqueous-two phase extraction,
(c) subjecting the separated target phase to diafiltration using a crossflow diafiltration unit,
   wherein the crossflow diafiltration unit at least comprises a diafiltration channel, a first filter material, a retentate channel, a second filter material and a permeate collection channel, arranged in such a way that the first filter material delimits the diafiltration channel and the retentate channel from one another, and the second filter material delimits the retentate channel and the permeate collection channel from one another,
   wherein the diafiltration channel is connected in a fluid conducting manner to at least one inlet for the diafiltration medium; the retentate channel is connected in a fluid conducting manner to at least one inlet for the target phase and to at least one outlet for the retentate;
   and the permeate collection channel is connected in a fluid conducting manner to at least one outlet for the permeate. The above statements and definitions analogously apply to this aspect of the present invention. Preferably, the method according to the present invention is carried out by using the separation system according to the present invention.

In a further aspect, the present invention relates to the use of a crossflow diafiltration unit for processing a target phase, which is obtained after separation of an aqueous two-phase system and contains a target component, wherein the crossflow diafiltration unit at least comprises a diafiltration channel, a first filter material, a retentate channel, a second filter material and a permeate collection channel, arranged in such a way that the first filter material delimits the diafiltration channel and the retentate channel from one another, and the second filter material delimits the retentate channel and the permeate collection channel from one another,
wherein the diafiltration channel is connected in a fluid conducting manner to at least one inlet for the diafiltration medium; the retentate channel is connected in a fluid conducting manner to at least one inlet for the target phase and to at least one outlet for the retentate;
and the permeate collection channel is connected in a fluid conducting manner to at least one outlet for the permeate. The above statements and definitions analogously apply to this aspect of the present invention. Preferably, the crossflow diafiltration unit is the crossflow diafiltration unit of the separation system according to the present invention.
Preferably, the aqueous two-phase extraction is carried out by applying the aqueous two-phase extraction unit of the separation system according to the present invention.

### Reference signs

Figures 1 and 3
   1 = light, polymer rich phase (LP), 2 = target component, mAb, 3 = host cell protein (HCP), 4 = cell, 5 = DNA, 6 = heavy salt rich phase (HP), 31 = target component, 32 = ATPS phase forming components, 33 = diafiltration buffer
Fig. 1: Schematic representation of the targeted distribution of biomolecules in the aqueous two-phase system for the extraction of, for example mAb as a target component.
Fig. 2: Schematic representation of an embodiment of the crossflow diafiltration unit used in the present invention. FIR= flow sensor (indicating, recording), CIR= conductivity sensor (indicating, recording), PIR= pressure sensor (indicating, recording).
Fig. 3: Schematic representation of an embodiment of the crossflow diafiltration unit used in the present invention during use. The ATPS target phase containing the target component as well as the phase forming components is supplied to the diafiltration unit via the feed inlet. Due to the selected MWCO of the membrane the target component is retained in the retentate channel, while the smaller phase forming components permeate through the membrane and are withdrawn in the permeate outlet. Simultaneously, the diafiltration buffer is actively supplied by an additional channel, permeates through the membrane and washes out the phase forming components from the retentate to the permeate (buffer exchange). The active supply of the diafiltration buffer strongly supports an efficient and fast buffer exchange resulting in significantly lower precipitation of the target component without prior dilution of the target phase.

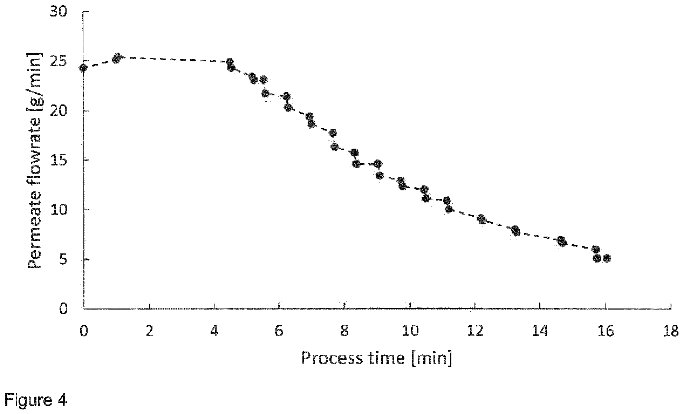

Fig. 4: Permeate flowrate throughout a conventional diafiltration of the target phase in Example 3.

The present invention will be further illustrated in the following examples without being limited thereto.

### Example 1

A mAb (immunoglobulin type G, IgG, isoelectric point=8.4) was produced in a fed-batch cultivation of CHO cells with a commercial serum-free medium. Cells were cultivated in an Ambr^{®}250 single use bioreactor (Sartorius, Göttingen, Germany) for 12 days at 855 rpm, 36.8 °C and pH 7.1. At harvest time point the viability was ≥ 80 % with a viable cell density ≥ 10*10⁶ cells/ml and an IgG concentration ≥ 2.8 g/l.

An ATPS composition (cf. Table 1) for a direct extraction from the cultivation broth was adopted from a previous study (Kruse et al. Antibodies 2019, 8, 40) based on a DoE approach for highest mAb yield and purity, regarding DNA and HCP, in the light target phase (LP). PEG with a molecular weight of 400 g/mol was purchased from Merck (Darmstadt, Germany). Sodium phosphate monobasic anhydrous (NaH₂PO₄) and potassium phosphate dibasic anhydrous (K₂HPO₄) were used for the preparation of the respective stock solutions (40 mass% phosphate buffer). The pH-value was adjusted by titration of both stock solutions. All salts including NaCl as displacement agent, were purchased from Carl Roth (Karlsruhe, Germany).

**Table 1. ATPS composition**

| **Component** | **Mass%** |
|---|---|
| **PEG400** | 19 |
| **40 wt.-% PO₄ buffer (pH 8.0)** | 41 |
| **Feed** | 36 |
| **NaCl** | 4 |

The appropriate amount of the ATPS components were weighed in a Schott flask. As feed cell containing cultivation broth was used. To ensure equilibrium conditions, the ATPS was stirred by a magnetic stirrer for 10 min at 500 rpm. For a quick and easy phase separation, centrifuge was used as a separation unit for separating the target phase (LP) obtained after aqueous-two phase extraction. Therefore, the ATPS was transferred into 500 ml Corning^{®} centrifuge tubes (Sigma-Aldrich, St. Louis, USA) and centrifuged for 5 min at 538 x g. The LP as mAb containing phase was withdrawn by pipetting. To ensure a complete cell removal, a sterile filter with a nominal pore size of 0.2 µm (Sartopore 2^{®} XLG, Sartorius, Göttingen) was used with a constant pressure of 1 bar for LP filtration.

**Table 2. Turbidity, concentration of the IgG as well as DNA and HCP and the respective amount in parts per million (ppm; related to the mAb concentration) obtained for the cell broth and the target phase after ATPE and phase separation.**

| **Parameter** | **Cell broth** | **Target phase** |
|---|---|---|
| **Turbidity [NTU]** | 2235 | 10.5 |
| **IgG concentration [mg/mL]** | 2.9 ± 0.2 | 2.2 ± 0.1 |
| **DNA concentration [µg/mL]** | 389 ± 3 | 16.8 ± 0.1 |
| **DNA content [ppm]** | 132936 | 7557 |
| **HCP concentration [µg/mL]** | 452 ± 27 | 228 ± 8 |
| **HCP content [ppm]** | 154565 | 102467 |

After ATPE and phase separation 81.9 % yield of mAb was obtained. The mAb was slightly diluted into the LP (2.2 mg/ml) compared to the concentration of the cultivation broth (2.9 mg/ml, Table 2). The turbidity decreased from 2235 to 10.5 NTU.

The majority of DNA (95.3 %) and 45.7 % of the HCP were removed after ATPE and phase separation as process related impurities. The DNA concentration was reduced from 393 µg/mL to 16.8 µg/mL, corresponding to 7557 ppm. Simultaneously, the HCP concentration was reduced from 452 µg/mL to 228 µg/mL corresponding to 102467 ppm (Table 2). These results demonstrate a sufficient clarification as well as product (IgG) capture directly from the cultivation broth by ATPE with high yield and simultaneous removal of process related impurities like DNA and HCP.

### Example 2

Such a separated target phase as described in Example 1 with the containing target component (IgG) was further purified by the diafiltration approach of the present invention. Therefore, a polyether sulfone membrane with mean molecular weight cut off of 30 kDa was used. Two different diafiltration buffers were exemplary examined: 50 mM KPi buffer, pH 7.4 as an example for neutral buffer conditions and 50 mM sodium acetate (NaAc), pH 3.0 as an example of an acidic buffer condition, which can also be used to enable a virus inactivation by acidification. For both diafiltrations 400 g of the separated target phase were processed, whereby the process can also be operated for a significantly higher amount of target phase or continuously.

**Table 3. Process parameters and results obtained for 50 mM KPi buffer, pH 7.4 as diafiltration buffer.**

| **50 mM KPi** | | **C IgG [g/L]** | **Yield [%]** |
|---|---|---|---|
| Target phase | 10 mL/min | | |
| Diafiltration buffer | 50 mL/min | | |
| Retentate | 10 mL/min | | |
| Processed target phase | 402.18 g or 359.1 mL | 2.033 | |
| Product | 325.6 g or mL | 2.108 | 94.0 |

**Table 4. Process parameters and results obtained for 50 mM sodium acetate, pH 3.0 as diafiltration buffer.**

| **50 mM NaAc** | | **C IgG [g/L]** | **Yield [%]** |
|---|---|---|---|
| Target phase | 10 mL/min | | |
| Diafiltration buffer | 50 mL/min | | |
| Retentate | 10 mL/min | | |
| Processed target phase | 402.27 g or 359.2 mL | 2.019 | |
| Product | 340.71 g or mL | 2.028 | 95.3 |

For both experiments, the flowrate of the target phase and retentate was set to 10 mL/min and the flowrate of the respective diafiltration buffer to 50 mL/min. Approximately 400 g were processed as planned corresponding to approximately 359 mL volume due to the density of the target phase. The IgG concentration of the product (retentate) was similar to the target phase with approximately 2 g/L due to the equal flowrate of both streams. For the entire process, a high IgG yield of 94.0 % (Table 3) and 95.3 % (Table 4) was achieved for the 50 mM KPi buffer and 50 mM sodium acetate, respectively. Throughout the entire process no precipitation was observed in the product phase and accordingly a very low turbidity below 5 NTU was obtained.

### Example 3

As a comparative example, the same target phase obtained by Example 1 was processed utilizing the same type of membrane as in Example 2 (30 kDa MWCO, polyether sulfone) but applying conventional diafiltration. A Sartoflow Smart benchtop diafiltration system with two Slice 200 membrane cassettes with a total membrane area of 400 cm² was used. As diafiltration buffer, similar to Example 2, 50 mM KPi buffer, pH 7.4 was used.

At the beginning of the diafiltration, a permeate flow of approximately 25 g/min was obtained, indicating an efficient buffer exchange. However, after already 4 minutes process time, the permeate flow strongly decreased to below 5 g/min, which made an abortion of the experiment necessary. Therefore, no complete diafiltration was possible in this case.

The membrane fouling was caused by a strong precipitation of the product (mAb), resulting in a high turbidity of approximately 500 NTU and a low yield of 48 % at the end of the aborted process.

A possible approach to enable a conventional diafiltration is to dilute the target phase five times with the diafiltration buffer to avoid precipitation of the target component as described by Kruse et al. 2020. However, this results in several drawbacks like longer processing times and the requirement for an additional concentration step to regain the initial concentration of the target component.

For this reason, the approach of the present invention offers several advantages, because no significant precipitation of the target component occurs, resulting in an efficient buffer exchange with high yields of the target component, and no additional dilution of the target phase is required.

## Claims

1. A separation system configured to process a fluid containing a plurality of components, wherein at least one component of the plurality of components of the fluid is a target component and wherein the separation system comprises:
an aqueous-two phase extraction unit for aqueous-two phase extraction of the fluid, a separation unit for separating a target phase obtained after aqueous-two phase extraction, wherein the target phase contains the target component, and
a crossflow diafiltration unit for continuous diafiltration of the separated target phase for obtaining a retentate and a permeate, wherein the crossflow diafiltration unit at least comprises a diafiltration channel, a first filter material, a retentate channel, a second filter material and a permeate collection channel, arranged in such a way that the first filter material delimits the diafiltration channel and the retentate channel from one another, and the second filter material delimits the retentate channel and the permeate collection channel from one another,
wherein the diafiltration channel is connected in a fluid conducting manner to at least one inlet for the diafiltration medium; the retentate channel is connected in a fluid conducting manner to at least one inlet for the target phase and to at least one outlet for the retentate; and the permeate collection channel is connected in a fluid conducting manner to at least one outlet for the permeate.

2. A method for purifying a target component included in a fluid, wherein the method comprises the steps of
(a) subjecting the fluid to aqueous two-phase extraction,
(b) separating a target phase containing the target component from the other phase obtained after aqueous-two phase extraction,
(c) subjecting the separated target phase to diafiltration using a crossflow diafiltration unit,
wherein the crossflow diafiltration unit at least comprises a diafiltration channel, a first filter material, a retentate channel, a second filter material and a permeate collection channel, arranged in such a way that the first filter material delimits the diafiltration channel and the retentate channel from one another, and the second filter material delimits the retentate channel and the permeate collection channel from one another,
wherein the diafiltration channel is connected in a fluid conducting manner to at least one inlet for the diafiltration medium; the retentate channel is connected in a fluid conducting manner to at least one inlet for the target phase and to at least one outlet for the retentate; and the permeate collection channel is connected in a fluid conducting manner to at least one outlet for the permeate.

3. Use of a crossflow diafiltration unit for processing a target phase, which is obtained after separation of an aqueous two-phase system and contains a target component,
wherein the crossflow diafiltration unit at least comprises a diafiltration channel, a first filter material, a retentate channel, a second filter material and a permeate collection channel, arranged in such a way that the first filter material delimits the diafiltration channel and the retentate channel from one another, and the second filter material delimits the retentate channel and the permeate collection channel from one another,
wherein the diafiltration channel is connected in a fluid conducting manner to at least one inlet for the diafiltration medium; the retentate channel is connected in a fluid conducting manner to at least one inlet for the target phase and to at least one outlet for the retentate; and the permeate collection channel is connected in a fluid conducting manner to at least one outlet for the permeate.

4. The separation system according to claim 1, or the method according to claim 2, or the use according to claim 3, wherein the target component is contained in the retentate after diafiltration.

5. The separation system according to claim 1 or 4, or the method according to claim 2 or 4, or the use according to claim 3 or 4, wherein the separated target phase is directly applied to the crossflow diafiltration unit.

6. The separation system according to any of claims 1, 4, and 5, or the method according to any of claims 2, 4, and 5, or the use according to any of claims 3 to 5, wherein a volume flow rate of the supplied diafiltration medium is 0.5 to 20 times the volume flow rate of the supplied target phase.

7. The separation system according to claim 6, or the method according to claim 6, or the use according to claim 6, wherein the volume flow rate of the supplied diafiltration medium is 5.0 to 7.0 times the volume flow rate of the supplied target phase.

8. The separation system according to any of claims 1 and 4 to 7, or the method according to any of claims 2 and 4 to 7, or the use according to any of claims 3 to 7, wherein the concentration of the target component in the separated target phase is from 0.5 g/L to 10 g/L.

9. The separation system according to any of claims 1 and 4 to 8, or the method according to any of claims 2 and 4 to 8, or the use according to any of claims 3 to 8, wherein the concentration of the target component in the retentate after diafiltration is from 0.5 g/L to 10 g/L.

10. The separation system according to any of claims 1 and 4 to 9, or the method according to any of claims 2 and 4 to 9, or the use according to any of claims 3 to 9, wherein the first filter material and the second filter material are identical.

11. The separation system according to any of claims 1 and 4 to 10, or the method according to any of claims 2 and 4 to 10, or the use according to any of claims 3 to 10, wherein the pH of the diafiltration medium is from 2.0 to 12.0.

12. The separation system according to any of claims 1 and 4 to 11, or the method according to any of claims 2 and 4 to 11, or the use according to any of claims 3 to 11, wherein the diafiltration medium is selected from the group consisting of KPi buffer, sodium phosphate buffer, sodium acetate buffer, PBS, glycine, citrate buffer, Tris buffer, BIS-Tris buffer, HEPES buffer, and water.

13. The separation system according to any of claims 1 and 4 to 12, or the method according to any of claims 2 and 4 to 12, or the use according to any of claims 3 to 12, wherein a composition for the aqueous-two phase extraction comprises, based on the total amount of the composition, from 4.0 to 25.0 wt.% of polyethylene glycol having an average molecular weight of from 300 to 20000; from 5.0 to 40 wt.% first salt selected from a phosphate salt, a citrate salt and a sulfate salt, or polymer; 0 to 10.0 wt.% second salt; 10.0 to 50.0 wt.% fluid containing the target component; and the balance being water.

14. The separation system according to any of claims 1 and 4 to 13, or the method according to any of claims 2 and 4 to 13, or the use according to any of claims 3 to 13, wherein the target component is selected from proteins, monoclonal antibodies, hormones, vaccines, nucleic acids, exosomes, viruses, and virus-like particles.
